# EUROPEAN PATENT APPLICATION

(11) **EP 4 534 669 A1**
(43) Date of publication of application: **09.04.2025**
(21) Application number: 23816322.4
(22) Date of filing: 30.05.2023
(51) Int. Cl.: C12N 15/11

(54) **NOVEL MODIFIED POLYADENINE SEQUENCE AND USE THEREOF**

(30) Priority: 31.05.2022 KR 20220066604
(71) Applicant: Industry-Academic Cooperation Foundation Gyeongsang National University, Jinju-si, Gyeongsangnam-do 52828 (KR); Korea Advanced Institute of Science and Technology, Guseong-dong, Yuseong-gu Daejeon 34141 (KR)
(72) Inventor: LEE, Kyuri, Jinju-si, Gyeongsangnam-do 52855 (KR); LEE, Young-suk, Yuseong-gu (KR); PARK, Minsa, Jinju-si, Gyeongsangnam-do 52826 (KR); KIM, Jin, Daegu 41165 (KR)
(74) Representative: Müller, Christian Stefan Gerd
(86) International application number: PCT/KR2023/007340
(87) International publication number: WO 2023/234661

(57) **Abstract**

The present invention relates to a modified poly A sequences and DNA sequences encoding the same that maintain its structural stability over an extended period within a biological sample. The modified poly-A sequence and the DNA sequences encoding the same according to the present invention possess an optimal full-length and regularly incorporate non-adenine (non-A) bases at appropriate positions, whereby the sequences are barely apt to decrease in length even within bacteria, ensuring a robust biological function as genetic material. Furthermore, the present invention may be beneficially utilized to stably produce a therapeutically effective amount of a target protein through an optimal poly A tail structure that enables the most efficient protein expression both in vivo and in vitro.

## Description

### Technical Field

The present invention relates to novel poly A sequences and DNA molecules encoding the same that have been modified to minimize reduction in length and maintain the structural stability within a biological sample over long periods.

### Background Art

Polyadenylation refers to the addition of a linear poly A polymer with repeated adenine bases to the terminus of an RNA molecule. In eukaryotic cells, polyadenylation is an important part in the cascade of events that leads to protein synthesis through transcription and translation. The poly A tail plays a key role in the release from the nucleus, translation, and stability of mRNA. As the length of the poly A tail shortens over time, the RNA molecule is degraded by enzymes, resulting in reduced or halted protein synthesis.

Therefore, the development of modified poly A sequences of which the length is maintained over an extended period or the shortening cycle of the full-length sequence is extended, thereby make the RNA molecule to maintain stability within various gene delivery vehicles or host cells, is increasingly in demand in fields of genetic engineering, recombinant production of target proteins, and genetic vaccines.

However, the DNA sequence encoding the poly A, the consecutive dA:dT base pairs, shortens rapidly in many bacteria, which poses many limitations in producing DNA constructs containing consecutive dA:dT by propagation in prokaryotic cells such as *E. coli.* Therefore, it is important to make appropriate modifications to the poly A-encoding DNA sequence to ensure that it remains efficiently stable when introduced into the bacteria via plasmid.

On the other hand, a genetic vaccine can be used for the prevention and treatment of cancer, infectious diseases, autoimmune diseases, and inflammatory diseases, etc. by using DNA or mRNA encoding an antigenic protein as an immunogen. Although DNA is known to be relatively stable and easy to handle compared to RNA for gene therapy, DNA has the disadvantage that when delivered into the genome of a subject, DNA can be inserted at unwanted locations resulting in damage of the host genes. DNA also may be damaged by anti-DNA antibodies produced by the host's immune response, and has limited expression levels of the target antigen protein due to various variables affecting transcription. In contrast, mRNA synthesizes proteins directly in the cytoplasm without transcription in the nucleus, does not risk damaging the genetic structure of the host cell, and does not induce long-term genetic modifications due to its short half-life, making it stable and easy to mass produce compared to DNA.

Therefore, the present inventors sought to identify a novel modified poly A-encoding DNA sequence that has a specific range of full-lengths and regularly contains non-adenine bases at appropriate locations, resulting in a long-lasting and dramatically increased expression efficiency of the target protein.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to explore the optimal structure of a modified poly A sequence or DNA sequence encoding the same, such that the full length of the poly A sequence in a biological sample, particularly in bacteria, remains stable over a long period of time, while the production efficiency of the target protein is significantly improved. As a result, the present inventors have found that when the polynucleotide encoding a poly A sequence comprising 70-230 adenine and non-adenine (non-A) bases, wherein the ratio between said adenine and non-adenine bases is 3:1 to 30:1, concretely 5:1 to 30:1, and more concretely 8:1 to 20:1, are used to produce the target protein, not only its length is maintained for a long period of time within a biological sample, e.g., a bacterium such as *E. coli.*, but also the expression efficiency of the target protein by the mRNA resulting from its transcription is dramatically improved.

Accordingly, it is an object of the present invention to provide a polynucleotide encoding a modified poly-adenyl sequence.

It is another object of the present invention to an RNA molecule comprising said modified polyadenine sequence or a DNA molecule encoding the same, and an immunogenic composition or pharmaceutical composition comprising it as an active ingredient.

It is another object of the present invention to a composition for modulating the expression and/or function of a target protein comprising an RNA molecule comprising said modified polyadenine sequence or a DNA molecule encoding the same, and an active ingredient thereof.

Other objects and advantages of the present invention will become more apparent from the following detailed description, the appended claims, and the accompanying drawings.

### Technical Solution

In one aspect of this invention, there is provided a polynucleotide that encodes a modified poly-adenyl sequence comprising 70 to 230 bases with a ratio of adenine to non-adenine (non-A) bases from 3:1 to 30:1.

The present inventors have made intensive studies to explore the optimal structure of a modified poly A sequence or DNA sequence encoding the same, such that the full length of the poly A sequence in a biological sample, particularly in bacteria, remains stable over a long period of time, while the production efficiency of the target protein is significantly improved. As a result, the present inventors have found that when the polynucleotide encoding a poly A sequence comprising 70-230 adenine and non-adenine (non-A) bases, wherein the ratio between said adenine and non-adenine bases is 3:1 to 30:1, concretely 5:1 to 30:1, and more concretely 8:1 to 20:1, are used to produce the target protein, not only its length is maintained for a long period of time within a biological sample, e.g., a bacterium such as *E. coli.*, but also the expression efficiency of the target protein by the mRNA resulting from its transcription is dramatically improved.

As used herein, the term "biological sample" refers to any sample containing a polynucleotide of the present invention, including, but not limited to, various eukaryotic cells, prokaryotic cells, tissues, organs, and cultures thereof. More specifically, said biological samples are cells and cultures thereof transformed with gene carriers containing polynucleotides of the present invention, more specifically prokaryotic cells or cultures thereof, and most specifically E. coli or cultures thereof.

As used herein, the term "gene delivery system" refers to a vehicle for introducing and expressing a desired target gene into a target cell. As used herein, the term "gene delivery" refers to the transport of a gene into a cell and has the same meaning as transduction of a gene into a cell. At the tissue and cellular level, "gene delivery" has the same meaning as spreading of a gene, and thus gene delivery vehicles may be described as gene penetration systems or gene diffusion systems.

As used herein, the term "nucleotide" refers to deoxyribonucleotide or ribonucleotide that exists in single-stranded or double-stranded form and includes nucleotide analogues with modified sugar or base, as well as natural-occurring nucleotides unless otherwise specifically noted (Scheit, Nucleotide Analogs, John Wiley, New York (1980); Uhlman and Peyman, Chemical Reviews, 90:543-584(1990)).

As used herein, the term "polyadenine sequence", "poly A sequence" or "poly(A) tail" refers to an adenine-repeating nucleotide sequence located at the 3' end of an RNA molecule that protects the RNA molecule from enzymatic degradation. The length of the poly A tail affects not only the stability of the mRNA, but also the translation of the protein.

According to a concrete embodiment, the non-adenine (non-A) base is located between 3 to 30 consecutive adenine sequences. More concretely, the non-A base is located between 3 to 20 consecutive adenine sequences, more concretely, between 3 to 15 consecutive adenine sequences, more concretely, between 3 to 10 consecutive adenine sequences, more concretely, between 5 to 10 consecutive adenine sequences, and most concretely, between 8 to 10 consecutive adenine sequences.

According to another concrete embodiment of the invention, the non-adenine base is located between 3, 5, 6, 8, 10, 15, 20 or 30 consecutive adenine sequences. There may be one non-adenine base between consecutive adenine sequences, or there may be multiple consecutive non-adenine bases. When a plurality of non-adenine bases are present between consecutive adenine sequences, there may be two or three consecutive non-adenine bases.

More concretely, one or two consecutive non-A bases are located between consecutive adenine sequences.

According to a concrete embodiment, where the same non-adenine base is applied, said non-adenine base may be Guanine, for example, (dG:dC) where the polynucleotide of the invention is a deoxyribonucleotide in double-stranded form.

According to a concrete embodiment, where two different non-adenine bases are applied, said non-adenine bases may be a combination of two or more bases selected from the group consisting of Guanine, Cytosine and Thymine, for example, (dG:dC), (dC:dG) and (dT:dA), respectively, where the polynucleotide of the invention is a deoxyribonucleotide in the double-stranded form. In this case, the two or more different non-adenine bases may be randomly spaced apart by a certain number of adenine bases, or they may be intersected. More concretely, it may be a combination of two or more bases selected from the group consisting of Guanine, Cytosine and Thymine.

According to a concrete embodiment, the ratio of adenine to non-adenine bases is from 5:1 to 30:1, and more concretely, 8:1 to 20:1.

According to another concrete embodiment of the invention, the ratio of adenine to non-adenine base is from 5:1 to 20:1, more concretely from 5:1 to 15:1, and more concretely from 5:1 to 10:1.

More concretely, the ratio of said adenine to non-adenine base is selected from the group consisting of 3:1, 5:1, 8:1, 10:1, 10:2, 10:3, 15:1, 15:2, 20:1, 30:1, and 30:2. Most concretely, the ratio of said adenine to non-adenine base is 8:1 or 10:1.

The number of single or consecutive non-adenine bases and the number of consecutive adenine bases present between them can be arranged in any form as long as the aforementioned content ratios (e.g., 3:1 to 30:1) are satisfied within the full-length sequence of the modified polyadenine of the present invention. Thus, the adenines and non-adenines are not necessarily repeated in the same ratio throughout the full-length sequence, and configurations in which they are repeated unevenly, for example, A(10) - non-A(1) - A(15) - non-A(2) - A(5) - non-A(1) - A(6) - non-A(2), such that the overall ratio (A : non-A) falls within the aforementioned content ratio range (6:1), are all included in aspects of the invention.

According to a concrete embodiment, the poly A sequences of the present invention comprise 74 to 190 bases, more concretely 80 to 190 bases, more concretely 90 to 190 bases, more concretely 100 to 190 bases, more concretely 109 to 186 bases, and most concretely 109 to 150 bases.

In another aspect of this invention, there is provided a DNA molecule comprising an expression regulatory sequence; a transducible nucleotide sequence operatively linked to the expression regulatory sequence; and a polynucleotide molecule encoding a modified polyadenine sequence of the present invention.

As used herein, the term " expression regulatory sequence" is meant to encompass an array of promoters, signaling sequences, and transcriptional regulator binding sites that are operatively linked to a target nucleic acid molecule to be expressed and regulate the initiation of expression thereof. More concretely, the expression regulatory sequence refers to a promoter.

As used herein, the term "promoter" refers to a regulatory nucleic acid sequence that directs the transcription of a nucleic acid and affects the expression of a target sequence to which it is operatively linked. A promoter may include a distal enhancer or repressor element, which may be arbitrarily located at a distance of several thousand base pairs from the transcription initiation site. The term "operatively linked" refers to a functional binding between an expression regulatory sequence and a target nucleic acid sequence, whereby the regulatory sequence regulates the transcription and/or decoding of the target nucleic acid molecule. According to a concrete embodiment, the expression regulatory sequence is selected from the group consisting of a T7 promoter, a T3 promoter and an SP6 promoter.

In still another aspect of this invention, there is provided an RNA molecule transcribed from the DNA molecule of the present invention described above.

According to a concrete embodiment, all or a portion of the uracil (U) is substituted with a modified U represented by following Formula 1: 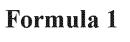 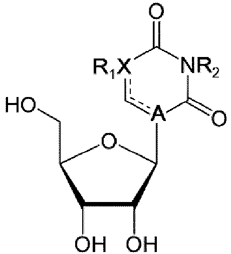 wherein R₁ and R₂ are each independently hydrogen, C₁-C₃ alkyl or C₁-C₃ alkoxy, X and A are carbon or nitrogen and are different from each other, and **- - -** is a single bond or a double bond.

As used herein, the term "alkyl" refers to a straight-chain or branched saturated hydrocarbon group, and includes, for example, methyl, ethyl, propyl, isopropyl, etc. C₁-C₃ alkyl refers to an alkyl group having an alkyl unit having 1 to 3 carbon atoms, and when the C₁-C₃ alkyl is substituted, the carbon atom number of the substituent is not included.

As used herein, the term "alkoxy" refers to a radical formed by the removal of hydrogen from an alcohol. When C₁-C₃ alkoxy is substituted, the number of carbons in the substituent is not included.

According to the octet rule, it is obvious that when X or A is nitrogen, **- - -** represents a single bond.

According to one embodiment, X is nitrogen, A is carbon, R₁ and R₂ are hydrogen. The compound represented by Formula 1 wherein X is nitrogen, A is carbon, and R₁ and R₂ are hydrogen is pseudouridine.

According to one embodiment, X is nitrogen, A is carbon, R₁ is C₁ alkyl (methyl) and R₂ is hydrogen. The compound represented by Formula 1 wherein X is nitrogen, A is carbon, R₁ is C₁ alkyl and R₂ is hydrogen is N1-methyl-pseudouridine.

According to one embodiment, X is carbon, A is nitrogen, R₁ is C₁ alkoxy (methoxy), and R₂ is hydrogen. The compound represented by Formula 1 wherein X is carbon, A is nitrogen, R₁ is methoxy, and R₂ is hydrogen is 5-methoxyuridine.

In still another aspect of this invention, there is provided an mRNA molecule comprising:
(a) a nucleotide sequence encoding a protein of interest; and
(b) a modified poly-adenyl sequence comprising 70 to 230 bases with a ratio of adenine to non-adenine (non-A) bases from 3:1 to 30:1.

The present may provide an mRNA molecule capable of stably expressing a target protein in a host cell by comprising an optimally modified poly A sequence. The modified poly A sequences of the present invention and the arrangement of the adenine and non-adenine bases therein have already been described above in detail and are therefore omitted to avoid undue redundancy.

One to three of the non-adenine (non-A) bases are positioned consecutively between consecutive adenine sequences, more concretely, one of the non-adenine (non-A) base is positioned between consecutive adenine sequences. As shown in the following Examples, the present inventors have found that the structure of poly A is more stable when two consecutive (m=2) non-adenine bases are introduced between the poly A sequences, but as for the transcribed IVT mRNA, the protein expression efficiency is higher when one (m=1) non-adenine base is introduced.

As used herein, the term "express" as used herein refers to being artificially replicated as an extrachromosomal factor or by chromosomal integration in a target cell via a gene delivery system to cause the target cell to express a exogenous gene or overexpress an endogenous gene. Accordingly, "express" may be used interchangeably with "transformation", "transfection", or "transduction".

According to a concrete embodiment, the nucleic acid sequence encoding the protein of interest comprises a 5'-UTR and a 3'-UTR joined at both ends.

More specifically, the 5'-UTR is bound to a 5'-cap.

As used herein, the term "untranslated region (UTR)" refers to an untranslated region within an mRNA that is bound to both ends of the coding sequence encoding the target protein. 5'-UTR and 3'-UTR are located upstream and downstream of the coding sequence, respectively. The term "5'-cap" refers to a component of an mRNA that is linked to the 5'-UTR and serves to bind the 40S ribosomal subunit to the mRNA by binding elF4E (eukaryote translation initiation factor 4E), which allows to initiate protein synthesis from the 5' initiation site of the mRNA, as well as to protect the mRNA from nucleases.

According to a concrete embodiment, The mRNA molecule of claim 11, wherein all or a portion of the uracil (U) is substituted with a modified U represented by following Formula 1:

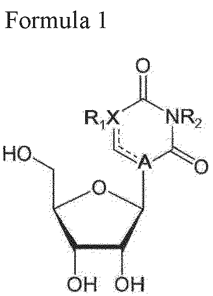

The modified bases in Formula 1 have already been described above in detail and are therefore omitted to avoid undue redundancy.

In still another aspect of this invention, there is provided a pharmaceutical composition comprising the DNA molecule, or the RNA molecule of the present invention as described above.

In still another aspect of this invention, there is provided a composition for modulating the expression and/or function of a protein of interest comprising the DNA molecule, or the RNA molecule of the present invention as described above.

In still another aspect of this invention, there is provided an immunogenic composition comprising the DNA molecule, the RNA molecule or the mRNA molecule of the present invention as described above.

As used herein, the term "immunogenic composition" refers to a composition that causes an adaptive immune response to a target antigen or immunogen expressed by the DNA or RNA molecule of the present invention, including, for example, a composition that causes the production of antibodies against the target antigen or immunogen after administered to a subject.

Such immunogenic compositions may be useful for preparation of vaccines for the prevention and/or treatment of disease, as well as for stably inducing autoantigen expression in non-human animals to create animal models to recapitulate an autoimmune disease, or for the production of therapeutic antibodies in host animals.

As used herein, the term "autoimmune disease" is meant to encompass any pathological condition in which a tissue or organ in the body is damaged or loses function due to an excessive or unwanted humoral or cellular immune response.

As used herein, the term "animal model" means a non-human animal that has been artificially modified, directly or indirectly, to have the traits, phenotype, or symptoms of a particular disease, such as an autoimmune disease, and more concretely, an animal into which an mRNA molecule of the present invention encoding an autoantigen protein has been introduced to recreate the overall biological process that proceeds by the production of autoantibodies.

As used herein, the term "antibody" refers to an immunoglobulin protein produced by the immune system of a mammal, that contains one or more variable domains that bind to and specifically recognizes an epitope of an antigen.

In still another aspect of this invention, there is provided a vaccine composition comprising the immunogenic composition of the present invention as described above as an active ingredient.

Immunogenic compositions comprising DNA molecules or RNA molecules of the present invention can be utilized as genetic vaccine compositions for various diseases that can be prevented, treated, or ameliorated through the immune response elicited thereby. As used herein, the term "vaccine" refers to a composition that eliminates or reduces a pathogenic substance or lesion tissue produced or introduced into the body through antibodies specific to the target antigen or immunogen produced by a patient's immune system by inducing a defensive immune response against the target antigen or immunogen, and is intended to include both prophylactic and therapeutic vaccines.

The present invention may be applied not only as a DNA vaccine but also as an mRNA-based vaccine. The mRNA can be mass-produced in 1-2 weeks only with a small amount of template DNA. *In vitro* production of mRNA does not require a biological reactor or direct handling of the infectious agent, thus making it easier to respond to rapidly spreading infectious diseases by rapid and safe synthesis of the antigen gene.

The term "preventing" as used herein refers to inhibiting the occurrence of a disorder or disease in a subject who has never been diagnosed as having the disorder or disease, but is at risk of developing the condition or disease.

The term "treating" as used herein refers to (a) inhibiting the progress of a disorder, disease or symptom; (b) alleviating the disorder, disease or symptom; or (c) eliminating the disorder, disease or symptom. When administered to a subject, the compositions of the present invention stably express the target antigenic protein, thereby efficiently inducing an immune response against it, which serves to block infection or to inhibit, eliminate, or alleviate symptoms thereof. The term "treating" as used herein refers to (a) inhibiting the progress of a disorder, disease or symptom; (b) alleviating the disorder, disease or symptom; or (c) eliminating the disorder, disease or symptom. When administered to a subject, the compositions of the present invention stably express the target antigenic protein, thereby efficiently inducing an immune response against it, which serves to block infection or to inhibit, eliminate, or alleviate symptoms thereof. Accordingly, the compositions of the present invention may be therapeutic vaccine compositions by themselves, or may be administered in combination with other pharmacologic agents as therapeutic adjuvants for the above diseases. Therefore, the term "therapeutics" or "therapeutic agent" includes the meanings of "therapeutic aid" or "therapeutic adjunct".

The term "administration" or "to administer" as used herein refers to the direct administration of a therapeutically effective amount of the composition of the present invention to a subject so that an equal amount is formed in the body of the subject.

The term "therapeutically effective amount" as used herein refers to the content of the composition of the present invention that is sufficient to provide a therapeutic or prophylactic effect to a subject to whom the composition is to be administered, and thus include the meaning of a "prophylactically effective amount".

The vaccine composition of the present invention is provided as a pharmaceutical composition, comprising a pharmaceutically acceptable carrier.

A pharmaceutically acceptable carrier contained the composition of the present invention is one commonly used in formulation. Examples of the pharmaceutically acceptable carrier include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present invention may further contain a lubricant, a wetting agent, a sweetening agent, a flavoring agent, an emulsifying agent, a suspending agent, a preservative, and the like, in addition to the above-described components. Suitable pharmaceutically acceptable carriers and agents are described in detail in Remington's Pharmaceutical Sciences (19th ed., 1995).

The pharmaceutical composition of the present invention may be administered parenterally, and concretely, intravenously, intramuscularly, subcutaneously, or intraperitoneally.

A suitable dosage of the pharmaceutical composition of the present invention may prescribed in a variety of ways depending on factors such as formulation method, administration mode, patient's age, weight, sex, pathological condition and diet, administration time, administration route, excretion rate, and response sensitivity. A preferred dosage of the pharmaceutical composition of the present invention is within the range of 0.001 to 100 mg/kg on an adult basis.

### Advantageous Effects

The features and advantages of the present invention are summarized as follows:
(a) The present invention provides a modified poly A sequences and DNA sequences encoding the same that maintain its structural stability over an extended period within a biological sample.
(b) The modified poly-A sequence and the DNA sequences encoding the same according to the present invention possess an optimal full-length and regularly incorporate non-adenine (non-A) bases at appropriate positions, whereby the sequences are barely apt to decrease in length even within bacteria, ensuring a robust biological function as genetic material.
(c) The present invention may be beneficially utilized to stably produce a therapeutically effective amount of a target protein through an optimal poly A tail structure that enables the most efficient protein expression both *in vivo* and *in vitro.*

### Brief Description of Drawings

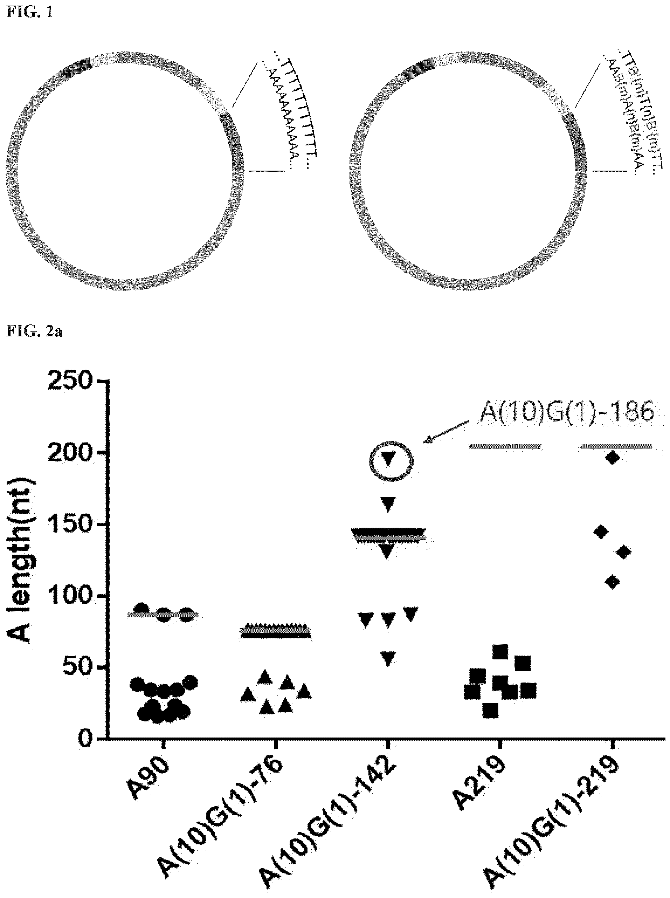

FIG. 1 shows a schematic of a DNA vector for IVT mRNA synthesis, where dark gray indicates the promoter region, light green indicates the untranslated region (UTR), blue indicates the coding sequence, and red indicates the poly(A) sequence. B indicates non-adenine and B' indicates DNA complementary to non-adenine. The left represents the vector DNA containing only pure poly(dA:dT) sequence, and the right represents the vector DNA with a non-adenine base (B) and its complementary base (B') introduced within the poly(dA:dT) sequence.

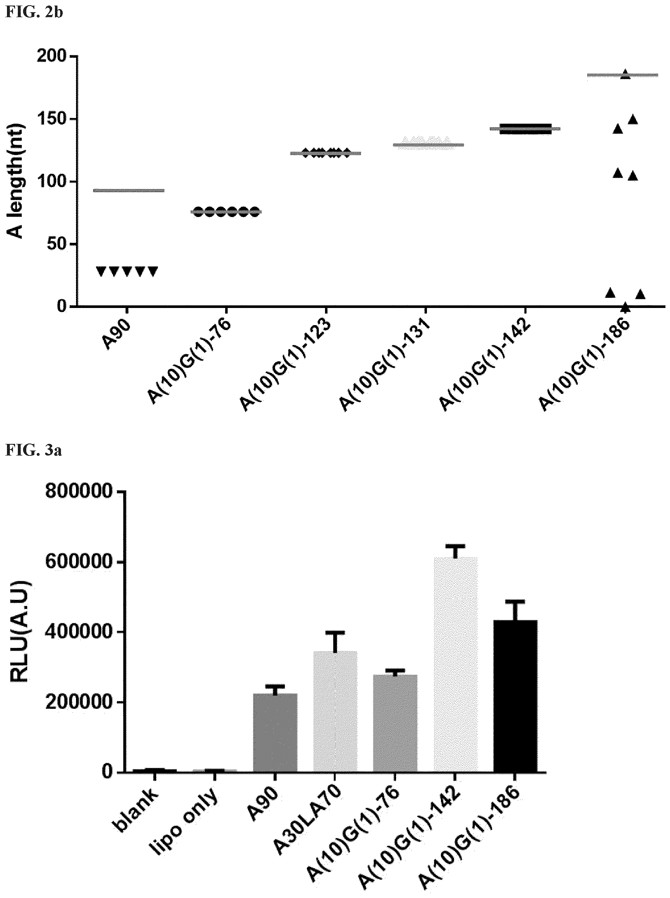

FIG. 2 shows the results of measuring the stability of the DNA vector *in E. coli* according to the full length of the poly (dA:dT) sequence (FIG. 2a) and the results of determining whether this stability is maintained after re-seeding of *E. coli* (FIG. 2b). The grey line in each graph represents stably maintained length of the poly (dA:dT) sequence introduced into the vector by cloning, and a dot is placed below the grey line when the poly(dA:dT) sequence length is shortened due to lack of stability.

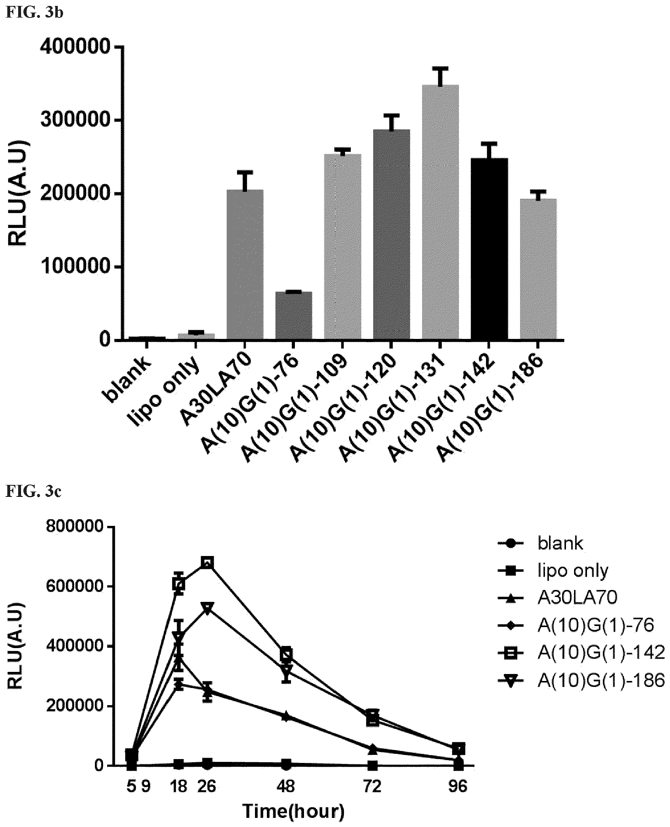

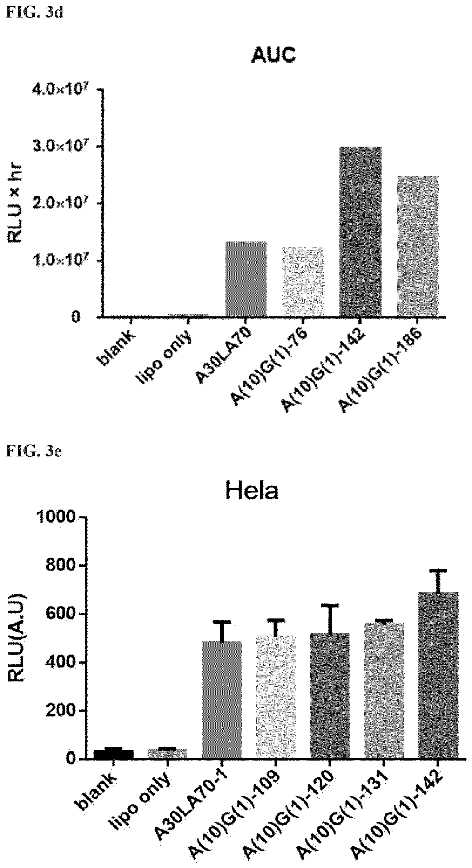

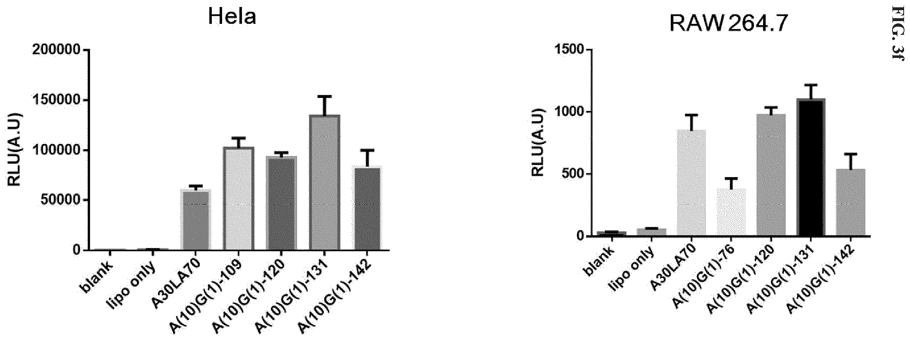

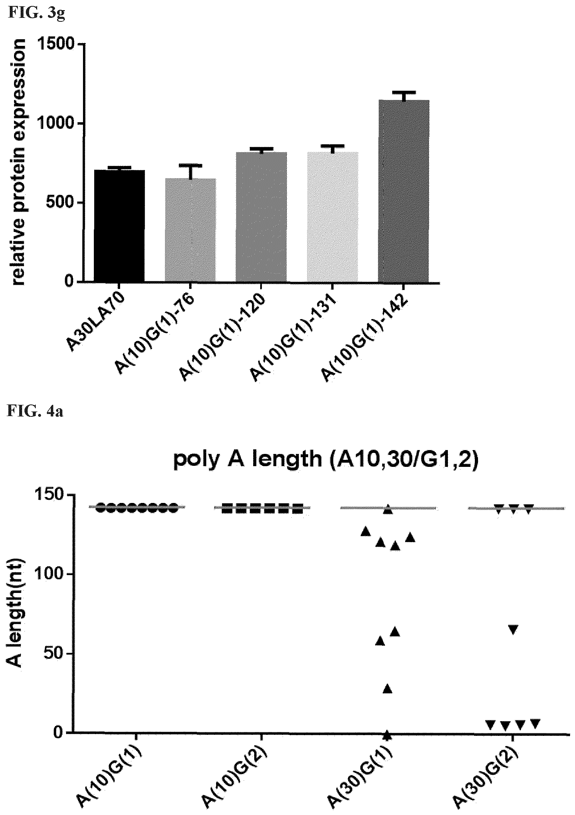

FIG. 3 shows the results of measuring protein expression efficiency based on *in vitro* transcribed (IVT) mRNA transcribed from vectors introducing various proportions of non-adenine bases and their complementary bases within poly(dA:dT) sequences of varying lengths. The measurement was carried out using mRNA containing the modified base (pUTP) at 18 hours (FIG. 3a), 24 hours (FIG. 3b), and each time point up to 96 hours (FIGs. 3c and 3d) through luciferase assays. FIG. 3e shows the results of measuring the efficiency of protein expression of unmodified IVT mRNA without the modified base (pseudouridine) using HeLa cells. FIG. 3f shows the results of measuring the efficiency of protein expression using HeLa cells and RAW 264.7 cells. FIG. 3g shows the results of measuring the protein expression efficiency of IVT mRNA containing the modified base (pUTP). FIG. 3g shows the results of measuring the protein expression efficiency of IVT mRNA containing the modified base (pUTP) that encodes EPO (erythropoietin). Each error bar represents the standard error of the mean.

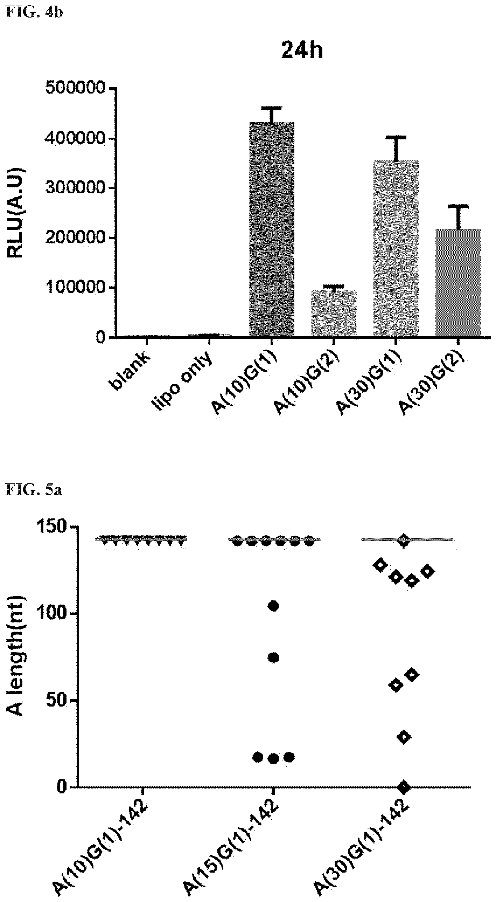

FIG. 4 shows the stability of poly(dA:dT) sequence constructs in *E. coli* where one or two non-adenine bases and their complementary bases are introduced into the poly(dA:dT) sequence (m=1, 2) (FIG. 4a), and the efficiency of IVT mRNA-based protein expression therefrom (FIG. 4b). The error bars in FIG. 4b represent the standard error of the mean.

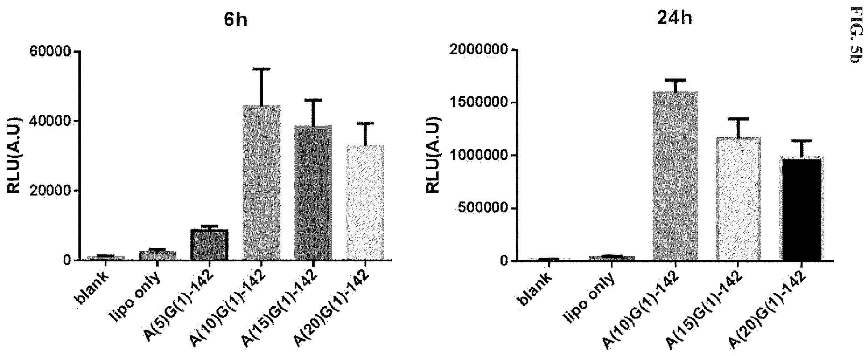

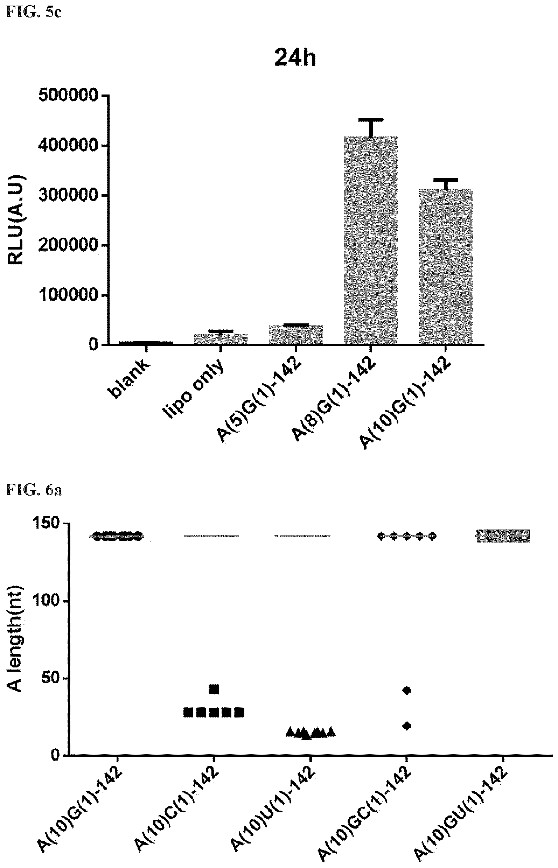

FIG. 5 represents the results of comparing the stability of poly(dA:dT) sequence structure (FIG. 5a) and the efficiency of IVT mRNA-based protein expression (FIGs. 5b and 5c) according to the distance between the inserted non-adenine (non-A) bases within the poly(dA:dT) sequence. The error bars in FIGs. 5b and 5c represent the standard error of the mean.

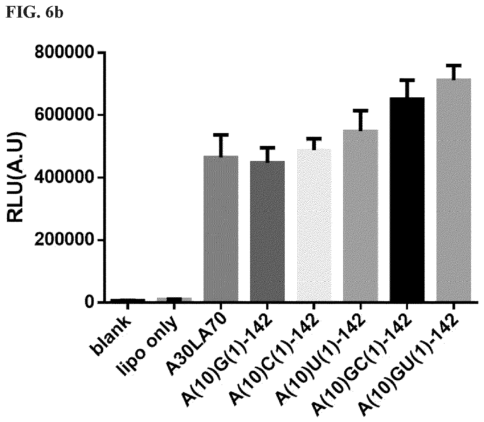

FIG. 6 illustrates a comparison for the structural stability of the poly(dA:dT) sequence (FIG. 6a) and the efficiency of IVT mRNA-based protein expression transcribed from it (FIG. 6b), depending on the type of non-A base inserted within the poly(dA:dT) sequence. A(10)GC(1)-142 denotes a structure of 142-full length in which two non-adenine bases, a single guanine and cytosine (m=1), are interspersed between ten adenine bases (n=10). A(10)GU(1)-142 denotes a structure of 142-full length with a single base (m=1) of guanine and uracil as non-adenine bases interspersed between 10 adenine bases (n=10). The error bars in FIG. 6b represent the standard error of the mean.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### Preparation of pDNA template for IVT mRNA synthesis and transformation into E.coli

To prepare the poly(dT:dA) inserted pDNA, the experiment was performed as follows. First, a DNA vector (luciferase-pcDNA3 or EPO-pcDNA3) containing the open reading frame (ORF) and 3'UTR coding for the model protein (firefly luciferase) or EPO (erythropoietin) was used as a template to perform PCR to construct the vector insert. PCR was performed using TOPsimple^{™} PCR DryMIX-Forte (enzynomics, Korea) under the conditions in Table 1 below, and the amplified PCR reaction products were purified using the AccuPrep^{®} PCR Purification Kit (Bioneer, Korea).

**[Table 1]**

| PCR Mixtures | | PCR cycles | | | |
|---|---|---|---|---|---|
| PCR Mixtures (FORTE) | 1 tube | Initial denaturation | 95 °C | 5 min | 1 cycle |
| DW | 17µl | denaturation | 95 °C | 30s | 30 cycles |
| pLuc (3~5 ng/µl) or pEPO | 1µl | Annealing | 59 °C | 30s | |
| FP (10 pmole/µl) | 1µl | Extension | 72 °C | 1min/kb | |
| RP (10 pmole/µl) | 1µl | Final Extension | 72 °C | 5min | 1 cycle |

The forward primer (FP) used contains 20-nt of the same sequence present at the end of the vector DNA being introduced for insertion into the future vector. The reverse primer (RP) containing the poly(T) sequence and 20-nt of the same sequence for insertion into the future vector was used to prepare a DNA template in which the poly(T) tail sequence and the same sequence were introduced in the 3' immediately adjacent to the 3'UTR. In other words, the forward and reverse primers were based on the following composition

*Forward primer1: 5'-homologous DNA sequence-a primer sequence that binds specifically to the DNA vector.*

*Reverse primer1: 5'-homologous DNA sequence - poly(T) - primer sequence that binds specifically to the DNA vector - 3'*

In the present invention, the primers comprising only poly(T) sequences and the primers in which one to three non-T(C/G) sequences were incorporated for every appropriate number of poly(T) sequences, were used as the poly(T) sequences in the reverse primers. This ensured that one to three different sequences were mixed into for every indicated interval of poly(dT:dA).

The insert prepared by the above method was inserted into a linear vector containing the 5'UTR sequence from T7 promoter. The linear vector was prepared by PCR using a DNA vector (GFP-pUC) as a template. The PCR was performed using TOPsimple^{™} PCR DryMIX-Forte (enzynomics, Korea), and the specific PCR conditions are shown in Table 1 above. The amplified PCR reactants were purified using the AccuPrep^{®} PCR Purification Kit (Bioneer, Korea).

In this case, the forward and reverse primers including the same 20-nt sequence at the end of the insert were used. In addition, the linear vector prepared thereby was made to include a 5'UTR sequence from the T7 promoter of the template DNA vector (GFP-pUC). In other words, the forward and reverse primers used for the preparation of the linear vector were based on the following configuration.

*Forward primer 2: 5'-homologous DNA sequence-a primer sequence that binds specifically to the DNA vector.*

*Reverse primer2: 5'-homologous DNA sequence-primer sequence that binds specifically to the DNA vector-3'*

The linear vector and the insert prepared by the above procedure were mixed in a 1:3 ratio and cloned by the SLIC (Sequence-& Ligation-Independent Cloning) method using T4 polymerase. The specific mixture conditions for SLIC are shown in Table 2 below. The mixture was reacted at room temperature for 2.5 min, followed by 10 min stabilization on ice to induce annealing of the linear vector and insert. The mixture was then transfected into competent cells (JUMBO Value 108 HIT-DH5α, RBCBioscience). The transfected competent cells were plated on solid media and incubated at 37°C for 16 hours.

After incubation, several single colonies were collected and subjected to colony PCR to confirm that the length of Poly(dT:dA) in the transformed vector was properly maintained in the colonies that formed. The forward primer and reverse primer of the colony PCR consisted of the forward and reverse sequences of Poly(dT:dA), so that the length of the amplification product of the colony PCR could be checked to evaluate whether the length of Poly(dT:dA) in the vector was stably maintained. A detailed method for verifying the length of Poly(dT:dA) is described in the following Examples.

**[Table 2]**

| | |
|---|---|
| Linear Vectors | a mole |
| Insert | b mole |
| 10x BSA | 1 µl |
| 10x Buffer | 1 µl |
| DW | up to 10µl |
| T4 polymerase (3 unit/µl) | 0.5 µl |

All primer sequences used in the present invention were obtained from IDT (Gene Synthesis Service), and are summarized in Table 3 below.

**[Table 3]**

| **Primer** | **Sequence** | **Sequence #** |
|---|---|---|
| Forward-Luciferase-pcDNA3 | 5'-AAATATAAGAgccaccatggaagacgc-3' | 4 |
| Reverse-Luciferase-pcDNA3 | 5’- TGATTTGCGTCGACACTAGTGGTCTCTTTTTTTTTTTTCTTTT TTTTTTCTTTTTTTTTTCTTTTTTTTTTCTTTTTTTTTTCTTTTT TTTTTCTTTTTTTTTTCAATGCGATGCAATTTCCTCATTTTA TTAGG-3’ | 5 |
| <A(10)G(1)-76> | | |
| Reverse-Luciferase-pcDNA3 | 5'- TGATTTGCGTCGACACTAGTGGTCTCTTTTTTTTTTTTCTTTT TTTTTTCTTTTTTTTTTTCTTTTTTTTTTTCTTTTTTTTTTTCTTTTT TTTTTCTTTTTTTTTTTCTTTTTTTTTTTCTTTTTTTTTTTCTTTTTT TTTTCTTTTTTTTTTTCTTTTTTTTTTTCTTTTTTTTTTTCAATGCG ATGCAATTTCCTCATTTTATTAGG-3' | 5 |
| <a(10)g(1)-142> | | |
| Reverse-Luciferase-pcDNA3 | 5'- TGATTTGCGTCGACACTAGTGGTCTCTTTTTTTTTTTTTCTTTT TTTTTTCTTTTTTTTTTTCTTTTTTTTTTTCTTTTTTTTTTTCTTTTTTT TTTTCTTTTTTTTTTTCTTTTTTTTTTTCTTTTTTTTTTTCTTTTTTTT TTTCTTTTTTTTTTTCTTTTTTTTTTTCTTTTTTTTTTTTTCAATGCG ATGCAATTTCCTCATTTTATTAGG-3' | 7 |
| <a(10)g(1)-186> | | |
| Reverse-Luciferase-pcDNA3 | 5'- TGATTTGCGTCGTCGACACTAGTGGTCTCTCTTTTTTTTTTTT TTTTGTTTTTTTTTTTGTTTTTTTTTTTGTTTTTTTTTTTGTTTTTTT TTTTGTTTTTTTTTTTGTTTTTTTTTTTGTTTTTTTTTTTGTTTTTTT [?] TTTTGTTTTTTTTTTTGTTTTTTTTTTTGTTTTTTTTTTTGTTTTTTT TTTTGTTTTTTTTTTTTCAATGCGATGCAATTTCCTCATTTTT ATTAGG-3' | 3 |
| <a(10)c(1)-142> | | |
| Reverse-Luciferase-pcDNA3 | 5'- TGATTTGCGTCGACACTAGTGGTCTCTTTTTTTTTTTTTTTTTTTTTTTTTC TTTTTTTTTTTTTTTCTTTTTTTTTTTTTTTTTTCTTTTTTTTTTTTTTT TTCTTTTTTTTTTTTTTTTTTCTTTTTTTTTTTTTTTTTTCTTTTTTTTTT TTTTTCTTTTTTTTTTTTTTTTTTCTTTTTTTTTTTTTTTTTTCAATGCG ATGCAATTTCCTCATTTTATTAGG-3' | |
| <a(15)g(1)-142> | | |
| Reverse-Luciferase-pcDNA3 | 5'- TGATTTGCGTCGACACTAGTGGTCTCTTTTTTTCCTTTTTTT TTTTTTTTCCTTTTTTTTTTTTTTTTCCTTTTTTTTTTTTTTTTCC TTTTTTTTTTTTTTTTCCTTTTTTTTTTTTTTTTCCTTTTTTTTTTTT TTTTCCTTTTTTTTTTTTTTTTCCTTTTTTTTTTTTTTTTCAATGC GATGCAATTTCCTCATTTTATTAGG-3' | 10 |
| <a(15)g(2)-142> | | |
| Reverse-Luciferase-pcDNA3 | 5'- TGATTTGCGTCGACACTAGTGGTCTCTTTTTTTTTTTTTTTT TCTTTTTTTTTTTTTTTTTTTTCTTTTTTTTTTTTTTTTTTTTCT TTTTTTTTTTTTTTTTTTTCTTTTTTTTTTTTTTTTTTTTCTTTT TTTTTTTTTTTTTTTTCTTTTTTTTTTTTTTTTTTTTCAATGCG ATGCAATTTCCTCATTTTATTAGG-3' | 11 |
| <a(20)g(1)-142> | | |
| Reverse-Luciferase-pcDNA3 | 5′- TGATTTGCGTCGACACTAGTGGTCTCTTTTTTTTTTTTTTTT TTTCTTTTTTTTTTTTTTTTTTTTTTTTTTTTTCTTTTTTTTTT TTTTTTTTTTTTTTTTTTTTTTTCTTTTTTTTTTTTTTTTTTTT TTTTTTCTTTTTTTTTTTTTTTTTTTTTTTTTTTTTCAATGCG ATGCAATTTCCTCATTTTATTAGG-3′ | 12 |
| <A(30)G(1)-142> | | |
| Reverse-Luciferase-pcDNA3 | 5’- TGATTTGCGTCGACACTAGTGGTCTCTTTTTTTTTTTTTTTTTTC CTTTTTTTTTTTTTTTTTTTTTTTTTTTTTTTTCCTTTTTTTTTT TTTTTTTTTTTTTTTTTTTTTCCTTTTTTTTTTTTTTTTTTTTTT TTTTTCCTTTTTTTTTTTTTTTTTTTTTTTTTTTTTTTCAATGCG ATGCAATTTCCTCATTTTATTAGG-3’ | 13 |
| <a(30)g(2)-142> | | |
| Reverse-Luciferase-pcDNA3 | 5′- TGATTTGCGTCGACACTAGTGGTCTCTTTTTCTTTTTCTTTT TCTTTTTCTTTTTCTTTTTCTTTTTCTTTTTCTTTTTCTTTTTC TTTTTCTTTTTCTTTTTCTTTTTCTTTTTCTTTTTCTTTTTCTT TTTCTTTTTCTTTTTCTTTTTCTTTTTCTTTTTCTTTTTCAATG CGATGCAATTTCCTCATTTTATTAGG-3′ | 14 |
| <A(5)G(1)-142> | | |
| Reverse-Luciferase-pcDNA3 | 5'- TGATTTGCGTCGACACTAGTGGTCTCTTTCTTTCTTTCTT TCTTTCTTTCTTTCTTTCTTTCTTTCTTTCTTTCTTTCTTTCTT TCTTTCTTTCTTTCTTTCTTTCTTTCTTTCTTTCTTTCTTTCTT TCTTTCTTTCTTTCTTTCTTTCTTTCTTTCTTTCTTTCTTTCTT TCTTTCTTTCTTTTCAATGCGATGCAATTTCCTCATTTTATT AGG-3' | 15 |
| <A(3)G(1)-142> | | |
| Reverse-Luciferase-pcDNA3 | 5'- TGATTTGCGTCGTCGACACTAGTGGTCTCTCTTTTTTTTTTT TTTTCCTTTTTTTTTTTTTCCTTTTTTTTTTTTTCCTTTTTTTTTT TTCCTTTTTTTTTTTTTCCTTTTTTTTTTTTTCCTTTTTTTTTTTT CCTTTTTTTTTTTTTCCTTTTTTTTTTTTTCCTTTTTTTTTTTTTCC TTTTTTTTTTTTTCAATGCGATGCAATTTCCTCATTTTTATTA GG-3' | 16 |
| <a(10)g(2)-142> | | |
| Reverse-Luciferase-pcDNA3 | 5'- TGATTTGCGTCGTCGACACTAGTGGTCTCTCTCCTTTTTTTT TTTTCCCTTTTTTTTTCCCTTTTTTTTTTTTCCCTTTTTTTTTTTTT CCCTTTTTTTTTTTTTTCCCTTTTTTTTTTCCCTTTTTTTTTTTTTT CCCTTTTTTTTTTTTTTTCCCTTTTTTTTCCCTTTTTTTTTTTTTTT TTTTTTCCCTTTTTTTTTTTTTTTTCAATGCGATGCAATTTCCTC ATTTTTATTAGG-3' | 17 |
| <a(10)g(3)-142> | | |
| Reverse-Luciferase-pcDNA3 | 5’- TGATTTGCGTCGACACTAGTGGTCTCTTTTTTTTTTTTCTTTT TTTTTTCTTTTTTTTTTTCTTTTTTTTTTTCTTTTTTTTTTTCTTTTT TTTTTCTTTTTTTTTTTCTTTTTTTTTTTCTTTTTTTTTTTCTTTTTT TTTTCAATGCGATGCAATTTCCTCATTTTATTAGG-3′ | 18 |
| <A(10)G(1)-109> | | |
| Reverse-Luciferase-pcDNA3 | 5′- TGATTTGCGTCGACACTAGTGGTCTCTTTTTTTTTTTTCTTTT TTTTTTCTTTTTTTTTTTCTTTTTTTTTTTCTTTTTTTTTTTCTTTTTT TTTTTCTTTTTTTTTTTCTTTTTTTTTTTCTTTTTTTTTTTCTTTTTTT TTTTCTTTTTTTTTTTCAATGCGATGCAATTTCCTCATTTTAT TAGG-3′ | 19 |
| <a(10)g(1)-120> | | |
| Reverse-Luciferase-pcDNA3 | 5′- TGATTTGCGTCGACACTAGTGGTCTCTTTTTTTTTTTCTTTT TTTTTTCTTTTTTTTTTTCTTTTTTTTTTTCTTTTTTTTTTTCTTTTT, TTTTTCTTTTTTTTTTTCTTTTTTTTTTTCTTTTTTTTTTTCTTTTTT TTTTCTTTTTTTTTTTCTTCAATGCGATGCAATTTCCTCATTT TATTAGG-3′ | 20 |
| <a(10)g(1)-123> | | |
| Reverse-Luciferase-pcDNA3 | 5′- TGATTTGCGTCGACACTAGTGGTCTCTTTTTTTTTTTTCTTTT TTTTTTCTTTTTTTTTTTCTTTTTTTTTTTCTTTTTTTTTTTCTTTTTT TTTTTCTTTTTTTTTTTCTTTTTTTTTTTCTTTTTTTTTTTCTTTTTTT TTTTCTTTTTTTTTTTCTTTTTTTTTTTCAATGCGATGCAATTTC CTCATTTTATTAGG-3′ | 21 |
| <A(10)G(1)-131> | | |
| Reverse-Luciferase-pcDNA3 | 5′- TGATTTGCGTCGACACTAGTGGTCTCTTTTTTTTTCTTTTTTTT ; TCTTTTTTTTTCTTTTTTTTTCTTTTTTTTTCTTTTTTTTTCTTTTTTT TTCTTTTTTTTTCTTTTTTTTTCTTTTTTTTTCTTTTTTTTTCTTTTTT TTTCTTTTTTTTTCTTTTTTTTTCTTTTTTTTTCTTTTTTTTTCAATG CGATGCAATTTCCTCATTTTATTAGG-3′ | 22 |
| <A(8)G(1)-142> | | |
| Reverse-Luciferase-pcDNA3 | 5'- TGATTTGCGTCGACACTAGTGGTCTCTTTTTTTTTTTATTTT TTTTTTATTTTTTTTTTATTTTTTTTTTATTTTTTTTTTATTTT TTTTTTATTTTTTTTTTATTTTTTTTTTATTTTTTTTTTATTTT TTTTTTATTTTTTTTTTATTTTTTTTTTATTTTTTTTTTCAATG CGATGCAATTTCCTCATTTTATTAGG-3' [?] | 23 |
| <A(10)U(1)-142> | | |
| Reverse-Luciferase-pcDNA3 | 5′- TGATTTGCGTCGACACTAGTGGTCTCTTTTTTTTTTTCTTTT TTTTTTGTTTTTTTTTTCTTTTTTTTTTGTTTTTTTTTTCTTTT TTTTTTGTTTTTTTTTTCTTTTTTTTTTGTTTTTTTTTTCTTTT TTTTTTGTTTTTTTTTTCTTTTTTTTTTGTTTTTTTTTTCAATG CGATGCAATTTCCTCATTTTATTAGG-3′ 2 | 24 |
| <a(10)gc(1)-142> | | |
| Reverse-Luciferase-pcDNA3 | 5′- TGATTTGCGTCGACACTAGTGGTCTCTTTTTTTTTTTTCTTTT TTTTTTATTTTTTTTTTTCTTTTTTTTTTTTATTTTTTTTTTTTCTTTT TTTTTTATTTTTTTTTTTCTTTTTTTTTTTTATTTTTTTTTTTTCTTTT 2 TTTTTTATTTTTTTTTTTCTTTTTTTTTTTTATTTTTTTTTTTCAATG CGATGCAATTTCCTCATTTTATTAGG-3′ | 25 |
| <a(10)gu(1)-142> | | |
| Reverse-Luciferase-pcDNA3 | 5′- TGATTTGCGTCGACACTAGTGGTCTCTTTTTTTTTTTTTTTTTT TTTTTTTTTTTTTTTTTTTTTTTTTTTTTTTTTTTTTTTTTTTTTTTTT 2 TTTTTTTTTAGTCATATGCTTTTTTTTTTTTTTTTTTTTTTTTTTTT TTTTTCAATGCGATGCAATTTCCTCATTTTATTAGG-3′ | 26 |
| <A30LA70> | | |
| Forward-GFP-UC | 5'-CTAGTGTCGACGCAAATCAGTTCT-3' | 27 |
| Reverse-GFP-UC | 5'-cCATGGTGGCTCTTATATTTCTTCTTCTTAC-3' | 28 |

### Verification of Poly(dA:dT) lengths

After transformation with the pDNA template prepared above, some of the single colonies cultured by spreading on solid medium were taken with a sterilized pipette tip and placed in a PCR tube for colony PCR. The forward and reverse primers used were composed of sequences that specifically bind to the sequences before and after the poly(dA:dT) sequence of the cloned pDNA. The PCR product was electrophoresed on a PAGE gel (15%) at 200 V for 40 min to determine the length of the poly(dA:dT) sequence. Furthermore, the length of the poly(dA:dT) sequence was reconfirmed by extracting the cloned pDNA and subjecting it to Sanger sequencing. *E.coli* transformed with the vector whose poly(dA:dT) length was confirmed by colony PCR and Sanger sequencing were stored as stocks in 50% glycerol.

### Maintenance of poly(dA:dT) length after reseeding of E.coli stocks transformed with pDNA containing poly(dA:dT).

To determine whether the poly(dA:dT) lengths were maintained after reseeding of the *E.coli* stock transformed with the vectors whose poly(dA:dT) lengths were identified, the stored *E.coli* were once again spread on solid media and incubated at 37°C for 16 hours. After incubation, a colony PCR was performed using primers specifically binding to the sequences before and after the poly(dT:dA) sequence to confirm that the poly(dA:dT) length in the transformed vector is properly maintained in the colonies. The length of the PCR product formed after the above colony PCR was electrophoresed on a PAGE gel (15%) at 200 V for 40 minutes to measure the length of the poly(dA:dT) sequence.

After liquid culture of the colonies, DNA was extracted and measured for the retention of poly(dA:dT) length by Sanger sequencing. As results, it was found that only three colonies of *E.coli* transformed with the vector containing A90 retained the poly(dA:dT) length, while none of the *E.coli* transformed with the vector containing A219 retained the poly(dA:dT) length. This suggests that as the length of the poly(dA:dT) increases, the stability of the DNA vector in *E.coli* decreases. On the other hand, in *E.coli* transformed with a vector containing a non-adenine base and its complementary base (dB:dB') inserted between the poly(dA:dT) sequences, the poly(dA:dT) sequence length was better maintained (FIG. 2a). Specifically, for A(10)G(1)-76, 75% of single colonies maintained stable poly(dA:dT) sequence length, and for A(10)G(1)-142, more than 80% of single colonies maintained stable poly(dA:dT) sequence length (FIG. 2a). However, for A(10)G(1)-219, the poly(dA:dT) sequence length was not maintained in all but one colony, indicating that the introduction of a single (dB:dB') between the poly(dA:dT) sequences decreased sequence structure stability when the total length of the poly(dA:dT) sequence exceeded 219 bases (FIG. 2a). From these results, it was found that the range of full length of poly(dA:dT) sequences that can maintain stability in *E.coli* when a single (dB:dB') is introduced between poly(dA:dT) sequences is 76 - 219 bases.

On the other hand, some colonies transformed with the vector cloned with A(10)G(1)-142 had poly(dA:dT) sequence lengths longer than those introduced poly(dA:dT) sequence length, suggesting that some of the primers used to clone the vector were longer than originally designed.

To further confirm that the poly(dA:dT) sequences of which the full-length and stability were confirmed would be still structurally stable in the *E.coli* stock, the present inventors measured the poly(dA:dT) sequence length in multiple single colonies formed after reseeding of the *E.coli* stock (FIG. 2b). The grey line in FIG 2b represents the poly(dA:dT) sequence length identified in the *E.coli* stock. When the poly(dA:dT) sequence length remained stable, the dots were located above the grey line, and when the poly(dA:dT) sequence length became shorter, the dots were located below the grey line. After liquid culture of the colonies, DNA was extracted and the poly(dA:dT) sequence length was confirmed by Sanger sequencing, which showed that the poly(dA:dT) sequence was perfectly maintained in A(10)G(1)-76, A(10)G(1)-123, A(10)G(1)-131, and A(10)G(1)-142 in the colonies formed after reseeding of *E. coli* (FIG. 2b). In case of A(10)G(1)-186, the length of the poly(dA:dT) was also observed to be maintained, indicating that when at least one (dB:dB') was introduced into the poly(dA:dT) sequence, stability was maintained up to the full length of the poly(dA:dT) sequence of 186 bases. In contrast, for A90, a pure poly(dA:dT) with no (dB:dB') insertions and a total sequence length of 90, no colonies retained the poly(dA:dT) length after reseeding. This confirmed that the poly(dA:dT) construct cannot be maintained in *E. coli* without the introduction of a non-adenine base pair.

### Preparation of IVT mRNA from each template pDNA

The pDNA template prepared above was used to synthesize IVT mRNA by *in vitro* transcription (IVT). To perform IVT, the pDNA prepared above was linearized with BsaI enzyme to be used as a template. Specifically, the mixture of pDNA and the enzyme was reacted at 37°C for 1 hour and then the enzyme was inactivated at 65°C for 20 minutes. The component of mixture for linearization of pDNA are shown in Table 4 below.

**[Table 4]**

| | |
|---|---|
| pDNA | 1 µg |
| 10x IV buffer | 1 µl |
| Bsa I (20unit/) | µl |
| DW | µl |
| total | µl |

Previously, all pDNAs were prepared with a BsaI enzyme site introduced at the 3' end of the poly(dA:dT) sequence. Therefore, the 3' end of the template DNA linearized by treatment with BsaI enzyme consists of various forms of poly(dA:dT) sequence.

Using the template DNA linearized by the above reaction, *in vitro* transcription was performed with a reaction solution containing T7 RNA polymerase, rATP, rCTP, rGTP, rUTP (or pseudouridine-5'-triphosphate) and a cap analogue (MEGAscript^{™} T7 Transcription Kit (Ambion, USA)). The template DNA was removed by DNase treatment and only mRNA was recovered. Specifically, the reaction solution was placed in a tube and incubated at 37°C for 2 hours, then 1 µl of Turbo DNaseI was added and reacted at 37°C for 15 minutes. The reaction product was purified using the RNeasy Mini Kit (Qiagen, USA). All components of the reaction solution, their dosages and concentrations are shown in Table 5 below. The mRNA synthesized thereby contained a 5'-cap at the 5' end, which was produced by addition of a cap analog to the transcription reaction.

**[Table 5]**

| | | |
|---|---|---|
| 3'-O-Me-m7G(5')ppp(5')G RNA Cap Structure Analog(NEB, USA) | 50mM | 1.6µl |
| rATP | 100mM | 1.0µl |
| rCTP | 100mM | 1.0µl |
| Pseudouridine-5'-Triphosphate(Trilink,USA) or rUTP | 100mM | 1.0µl |
| rGTP | 100mM | 0.2µl |
| 10X reaction buffer | | 2µl |
| DNA template(Enzyme cut) | 1µg | Xµl |
| DEPC | | Up to 20µl |
| 10X T7 polymerase | | 2µl |
| total | | 20µl |

### Evaluation of protein expression efficacy of the IVT mRNA

To determine the ability of IVT mRNAs with different poly(A) tails to express protein, HEK 293T cells, Hela cells, or RAW 264.7 cells were seeded in 96-well plates at 2.5 x 10⁴ (HEK 293T cells), 1 x 10⁴ (Hela cells), and 2 x 10⁴ (RAW 264.7 cells) per well, and cells were transfected with each IVT mRNA at a dose of 70 ng mRNA/well using transfection reagent (Lipofectamine^{™} 2000, Invitrogen, USA). The efficiency of intracellular firefly luciferase expression by time after transfection was measured with a luciferase assay system (Bright-Glo^{™} Ruciferase Assay System, Promega, USA). In addition, to measure the expression of erythropoietin (EPO) after transfection of IVT mRNA encoding EPO, cell culture media were collected 24 hours after transfection and subjected to ELISA assay using the Human EPO ELISA Kit (invitrogen, BMS2035-s).

The poly(A) sequence of the IVT mRNA constructed using a vector template containing (dB:dB') insertions between poly(dA:dT) sequences, will have the corresponding (dB:dB') number of non-adenine bases inserted. In addition, A30LA70, an IVT mRNA with a hybridized sequence of 10 non-adenines and adenines inserted between consecutive 30 adenines and 70 adenines, was used as a positive control.

### Expression of IVT mRNA containing modified bases (pUTP) in 293T cells

As shown in FIG. 3a, after 18 hours, the protein expression capacity of IVT mRNA containing the modified poly(A) sequence along with the modified base pUTP (pseudouridine) was found to be equivalent or superior to A30LA70, which has been reported as a successful poly(A) sequence in the art. In particular, IVT mRNAs from this outbreak with one non-adenine interspersed between consecutive adenine sequences and with a total length of 76-186 in the poly(A) tail were equivalent (A(10)G(1)-76) or significantly superior (A(10)G(1)-142 and A(10)G(1)-186) to A30LA70.

For a more precise exploration of the optimal total length of the poly(A) tail, the present inventors included A(10)G(1)-109; A(10)G(1)-120; and A(10)G(1)-131 as additional modified poly(A) sequences and analyzed luciferase expression after 24 hours using the same process as described above. As results, it was found that the total length of the poly(A) tail ranged from 109 to 186, significantly outperforming the positive control A30LA70 (FIG. 3b).

The protein expression of IVT mRNA with the modified poly(A) sequence was measured at different time points (5, 9, 18, 26, 48, 72 and 96 hrs), which further confirmed that the protein expression of the modified poly(A) sequence of the present invention was equal or superior to that of the positive control, A30LA70, consistently at various measurement time points (FIG. 3c), and the area under the curve (AUC) values for luciferase expression at different time points are shown in FIG. 3d.

### Expression of unmodified IVT mRNA in Hela and 293T cells

To further validate the change in expression efficiency caused by the inclusion of the modified bases, Hela cells were transfected with 0.7 µg/ml of various lengths of IVT mRNA without pUTP using Lipofectamine 2000 and measured protein expression 24 hours later. The results showed excellent expression efficiency in Hela cells, which was equivalent or superior to the positive control, A30LA70, at all lengths (FIG. 3e). This showed that IVT mRNA with the modified poly(A) tail of the present invention has a significantly superior protein expression efficiency even without the application of a modified base such as pseudouridine, and that the full-length of the poly(A) tail of 109-186 nucleotides is equivalent or better than that of A30LA70.

### Modified IVT mRNA in Hela and RAW 264.7 cells

To further validate the host cell-dependent variation in expression efficiency Hela cells and RAW 264.7 cells were transfected with 0.7 µg/ml of various lengths of modified IVT mRNA containing pUTP using Lipofectamine 2000 and measured protein expression 24 hours later. As shown in FIG. 3f, high luciferase expression levels were observed for all lengths, especially when the total length of the poly(A) tail was 109-186, which was equivalent to or greater than that of A30LA70. From these results, it was found that the modified poly(A) tail-transfected IVT mRNA of the present invention consistently exerts excellent efficacy in a variety of cells without cell type-specific expression deviations.

### EPO Expression by Modified IVT mRNA

To further validate the variation in expression efficiency depending on the type of target protein, Hela cells were transfected with 0.7µg/ml of the IVT mRNAs encoding EPO (erythropoietin) using Lipofectamine 2000 and analyzed the relative protein expression of each poly(A) construct 24 hours later. As shown in FIG. 3g, all lengths tested showed high EPO expression levels, especially A(10)G(1)-76 was equivalent, and 'A(10)G(1)-120, A(10)G(1)-131 and A(10)G(1)-142 were significantly superior, to A30LA70.

### Stability and protein expression efficiency According to the number of non-adenine bases

The present inventors sought to determine the stability of poly(dA:dT) sequence structures in *E. coli* when non-adenine bases and their complementary bases (dB:dB') were introduced by one (m=1) or two in succession (m=2) for every 10 to 30 poly(dA:dT) sequences. Sanger sequencing was performed on DNA extracted from cultures of multiple single colonies formed after reseeding of *E. coli* stocks. As results, it was found that the poly(dA:dT) sequence remained more stable when (dB:dB') was introduced one by one (m=1) than when two were introduced in succession (m=2) (FIG. 4a). Specifically, the structure of A(30)G(2) was more stable compared to A(30)G(1).

Furthermore, the efficiency of protein expression of IVT mRNA according to the number of (dB:dB') insertions within the poly(dA:dT) sequence were evaluated. The poly(A) sequence of the IVT mRNA prepared by using the vector with one or two consecutive (dB:dB') insertions between the poly(dA:dT) sequences as a template will have a corresponding number of (dB:dB') non-adenine bases inserted. The luciferase assays showed that the protein expression efficiency of IVT mRNA was higher when two (dB:dB') were introduced one by one (m=1) than when two (dB:dB') were introduced consecutively (m=2) (FIG. 4b). This result shows that the vector with two (dB:dB') introduced consecutively (m=2) has a higher structural stability for poly(dA:dT) *in E. coli,* but the protein expression efficiency of the IVT mRNA produced from this template is lower in m=2 compared to m=1.

### Stability and protein expression efficiency according to the distance between inserted non-adenine bases

The present inventors sought to evaluate the stability of poly(dA:dT) sequence constructs *in E. coli* according to the sequence length (n=10, 15, 30) of consecutive poly(dA:dT) sequences between the non-adenine base inserted, i.e. (dB:dB'). The poly(dA:dT) sequence lengths in multiple single colonies formed after re-seeding of *E. coli* stocks into solid media were determined by Sanger sequencing, As results, it was found that the stability decreased with increasing sequence length of consecutive poly(dA:dT) positioned between (dB:dB') in a poly(dA:dT) of the same overall length (FIG. 5a).

In addition, the efficiency of protein expression of the corresponding IVT mRNA according to the sequence length (n=5, 10, 15, 20) of consecutive poly(dA:dT) sequences between (dB:dB') inserted were compared. The luciferase assay showed that the presence of one (dB:dB') in the poly(dA:dT) sequence (m=1) efficiently expressed the protein when 5≤n≤20, with the highest protein expression at n=10 (FIG. 5b).

For a more specific exploration of the optimal length of the poly(dA:dT) sequences inserted between the non-adenine sequences, the luciferase expression after 24hr was further analyzed using the same process as described above for A(5)G(1)-142; A(8)G(1)-142; and A(10)G(1)-142. As results, it was found that A(8)G(1)-142 with n=8 showed the highest protein expression, indicating that protein expression is maximized when 5≤n≤10 (FIG. 5c).

### Stability and protein expression efficiency according to the type of non-adenine base

To evaluate the stability of the poly(dA:dT) sequence *in E. coli* according to the type of non-adenine base introduced between the adenine bases in the modified poly(A) constructs, the full length of the poly(dA:dT) sequence and the maintenance of the poly(dA:dT) sequence length in multiple single colonies formed after re-seeding of the stable *E. coli* stocks were measured (FIG. 6a). The grey line in FIG. 6a indicates the length of the poly(dA:dT) sequence identified in the *E. coli* stock. When the poly(dA:dT) sequence length identified after colony PCR of colonies formed after re-seeding of the *E. coli* stock is stable, the dot is located above the grey line, and when the poly(dA:dT) sequence length is not stable and shortens, the dot is located below the grey line. The poly(dA:dT) sequence length was reconfirmed by liquid culture of the colonies, followed by DNA extraction and sanger sequencing. As a result, it was observed that constructs with a batch of (dG:dC) introduced as a non-adenine base and its complementary base in the middle of the adenine base in the poly(dA:dT) sequence showed high stability, while constructs with a batch of (dC:dG) or (dT:dA) did not improve the stability of the poly(dA:dT) sequence. We found that when introducing one type of non-adenine base and its complementary base into poly(dA:dT), (dG:dC) can efficiently stabilize the poly(dA:dT) sequence. However, it was confirmed that the reduced stability of poly(dA:dT) sequence structures caused by the uniform introduction of (dC:dG) or (dT:dA) can be restored by alternating the placement of non-adenine base with (dC:dG) and (dG:dC) or with (dT:dA) and (dG:dC). This indicates that when introducing (dC:dG) or (dT:dA) into poly(dA:dT) as non-adenine bases, incorporation of (dG:dC) can improve vector stability. This suggests that when introducing non-adenine bases to improve the stability of poly(dA:dT) sequence, the type and pattern of introduction may affect the stability of the poly(dA:dT) sequence.

Luciferase assays were performed to compare the efficiency of protein expression when non-adenine bases and their complementary bases (dB:dB') were introduced one by one (m=1). Within the poly(A) tail structure of IVT mRNA transcribed from a template vector containing a modified poly(dA:dT) sequence, single non-adenine bases are intermittently inserted. Using the IVT mRNA designed to encode luciferase, the expression was analyzed. As results, IVT mRNA with a single non-adenine base of G, C, or U inserted between adenine bases in the poly(A) sequence was found to exhibit similar levels of expression. However, when non-adenine bases G/C or G/U were alternately introduced, the expression efficiency was observed to improve further compared to IVT mRNA with a single type of non-adenine base uniformly introduced (FIG. 6b). This confirms that introducing different types of non-adenine bases into the poly(dA:dT) sequence, rather than a single base, further improves protein expression.

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. A polynucleotide that encodes a modified poly-adenyl sequence comprising 70 to 230 bases with a ratio of adenine to non-adenine (non-A) bases from 3:1 to 30:1.

2. The polynucleotide of claim 1, wherein the non-adenine (non-A) base is located between 3 to 30 consecutive adenine sequences.

3. The polynucleotide of claim 1, wherein 1 to 3 of the non-adenine (non-A) bases are positioned consecutively between the consecutive adenine sequences.

4. The polynucleotide of claim 3, wherein 1 or 2 of the non-adenine (non-A) bases are positioned consecutively between the consecutive adenine sequences.

5. The polynucleotide of claim 1, wherein the ratio of adenine to non-adenine bases is from 5:1 to 30:1.

6. The polynucleotide of claim 5, wherein the ratio of adenine to non-adenine bases is selected from the group consisting of 3:1, 5:1, 8:1, 10:1, 10:2, 10:3, 15:1, 15:2, 20:1, 30:1, and 30:2.

7. A DNA molecule comprising:
an expression regulatory sequence;
a transducible nucleotide sequence operatively linked to the expression regulatory sequence; and
a polynucleotide molecule encoding a modified polyadenine sequence of any one of claims 1 to 6.

8. The DNA molecule of claim 7, wherein the expression regulatory sequence is selected from the group consisting of a T7 promoter, a T3 promoter, and an SP6 promoter.

9. An RNA molecule transcribed from the DNA molecule of claim 7.

10. The RNA molecule of claim 9, wherein all or a portion of the uracil (U) is substituted with a modified U represented by following Formula 1: 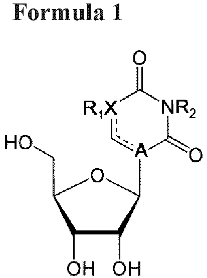 wherein R₁ and R₂ are each independently hydrogen, C₁-C₃ alkyl or C₁-C₃ alkoxy, X and A are carbon or nitrogen and are different from each other, and **- - -** is a single bond or a double bond.

11. An mRNA molecule comprising:
(a) a nucleotide sequence encoding a protein of interest; and
(b) a modified poly-adenyl sequence comprising 70 to 230 bases with a ratio of adenine to non-adenine (non-A) bases from 3:1 to 30:1.

12. The mRNA molecule of claim 11, wherein the non-adenine (non-A) base is located between 3 to 30 consecutive adenine sequences.

13. The mRNA molecule of claim 11, wherein 1 to 3 of the non-adenine (non-A) bases are positioned consecutively between the consecutive adenine sequences.

14. The mRNA molecule of claim 13, wherein 1 or 2 of the non-adenine (non-A) bases are positioned consecutively between the consecutive adenine sequences.

15. The mRNA molecule of claim 11, wherein the ratio of adenine to non-adenine bases is from 5:1 to 30:1.

16. The mRNA molecule of claim 15, wherein the ratio of adenine to non-adenine bases is selected from the group consisting of 3:1, 5:1, 8:1, 10:1, 10:2, 10:3, 15:1, 15:2, 20:1, 30:1, and 30:2.

17. The mRNA molecule of claim 11, wherein all or a portion of the uracil (U) is substituted with a modified U represented by following Formula 1: 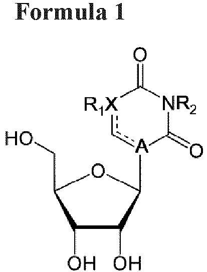 wherein R₁ and R₂ are each independently hydrogen, C₁-C₃ alkyl or C₁-C₃ alkoxy, X and A are carbon or nitrogen and are different from each other, and **- - -** is a single bond or a double bond.

18. An immunogenic composition comprising the DNA molecule of claim 7, the RNA molecule of claim 9, or the mRNA molecule of claim 11.

19. A vaccine composition comprising the immunogenic composition of claim 18 as an active ingredient.
